# EUROPEAN PATENT SPECIFICATION

(11) **EP 3 576 797 B1**
(45) Date of publication and mention of the grant of the patent: **03.07.2024**
(21) Application number: 18701599.5
(22) Date of filing: 18.01.2018
(51) Int. Cl.: A61L 2/07, A61L 11/00

(54) **STERILIZING APPARATUS FOR THE STERILIZATION OF POST-CONSUMER ABSORBENT SANITARY PRODUCTS**
STERILISATIONSVORRICHTUNG ZUR STERILISATION VON SAUGFÄHIGEN SANITÄRPRODUKTEN NACH GEBRAUCH
APPAREIL DE STÉRILISATION POUR LA STÉRILISATION DE PRODUITS SANITAIRES ABSORBANTS APRÈS UTILISATION

(30) Priority: 06.02.2017 IT 201700012621
(43) Date of publication of application: 11.12.2019
(73) Proprietor: The Procter & Gamble Company, Cincinnati, OH 45202 (US)
(72) Inventor: SOMMA, Marcello, 65010 Spoltore (PE) (IT); VACCARO, Giorgio, 65010 Spoltore (PE) (IT); PAGOTTO, Amedeo, 65010 Spoltore (PE) (IT)
(74) Representative: Parthey, Matthias
(86) International application number: PCT/IB2018/050304
(87) International publication number: WO 2018/142234

(56) References cited:
- EP-A1- 0 536 098
- WO-A1-93/06418
- WO-A1-2010/065088
- WO-A1-2012/049482
- WO-A2-2011/092509
- CN-U- 205 316 913
- DE-A1- 3 238 434

## Description

### Field of the invention

The present invention relates to a sterilizing apparatus, in particular for sterilizing post-consumer absorbent sanitary products

The term "absorbent sanitary products" generally refers to disposable absorbent products, such as diapers for babies, incontinence pads for adults, sanitary towels, absorbent tampons, absorbent bed linings, etc.

### Description of the prior art

Absorbent sanitary products are generally composed of a variety of different materials, including sheets of plastic material, cellulose fluff, superabsorbent polymers, non-woven sheets, etc.

Absorbent sanitary products contain high-quality materials such as plastics and cellulose and it is, therefore, desirable to recover these materials for use in the market of recycled raw materials or for producing energy.

One of the difficulties encountered in recycling post-consumer absorbent sanitary products is that these products contain organic excretions and bacteria, and it is therefore necessary to carry out sterilization of the products before separating the materials. Another difficulty derives from the fact that the used absorbent sanitary products are usually thrown folded on themselves and closed in a packet form, with the outer plastic layer of the products forming an impermeable barrier. The outer waterproof layer prevents effective sterilization of the products.

The document WO 2010/065088 describes an autoclave for treating solid urban waste, which envisages the drying of the waste by steam. The apparatus described in the document WO 2010/065088 comprises a cylindrical rotating autoclave equipped with at least one door, which can be opened to allow access inside the autoclave, and sealed closed to allow pressurization of the autoclave, an inlet for contact steam that comes into direct contact with the waste contained within the autoclave, and a plurality of hollow metal bars with a rectangular cross-section fed with non-contact steam. This apparatus allows the sterilization of solid urban waste and the drying of the waste during autoclaving.

EP-A-2596810 - by the same applicant - describes a method for treating used absorbent sanitary products, which envisages: loading post-consumer absorbent sanitary products into a cylindrical rotating autoclave having an inner surface, and heating and pressurizing the autoclave at a sterilization temperature and at the same time rotating the autoclave about a longitudinal axis thereof. Autoclave heating provides a first temperature regime for the products contained in the autoclave and a second temperature regime, higher than the first temperature regime, for the inner surface of the autoclave.

The heat required for reaching the temperature for sterilizing the products contained in the autoclave comes mostly from the heat energy transferred through the inner wall of the autoclave. If the heat exchange between the wall of the autoclave and the absorbent sanitary products is not optimal, with the same thermal energy available, the transfer time of the thermal energy to the products is high, and consequently the time of the sterilization cycle of the products is high.

WO 2012/049432 A1 discloses an autoclave for waste treatment comprising a rotating vessel, an out pressure container and helical tubes forming a cage around the internal container.

WO 93/06413 A1 and CN 205 316 913 U disclose sterilization apparatus for waste comprising a container housing, a steam generator and an auger. The waste is transported within the container by the auger and the steam is directly injected inside the container.

### Object and summary of the invention

The present invention aims to provide an apparatus for sterilizing post-consumer absorbent sanitary products that allows optimization of the transfer of thermal energy from the walls of the apparatus to the products.

According to the present invention, this object is achieved by an apparatus having the characteristics forming the subject of claim 1.

The claims form an integral part of the disclosure provided here in relation to the invention.

### Brief description of the drawings

The present invention will now be described in detail with reference to the attached drawings, given purely by way of non-limiting example, wherein:
- Figure 1 is an axial cross-section of an apparatus according to the present invention,
- Figure 2 is a diagram illustrating the steam supply to the apparatus of Figure 1, and
- Figures 3, 4 and 5 are details on an enlarged scale of the parts indicated by the arrows III, IV and V in Figure 1.

### Detailed description

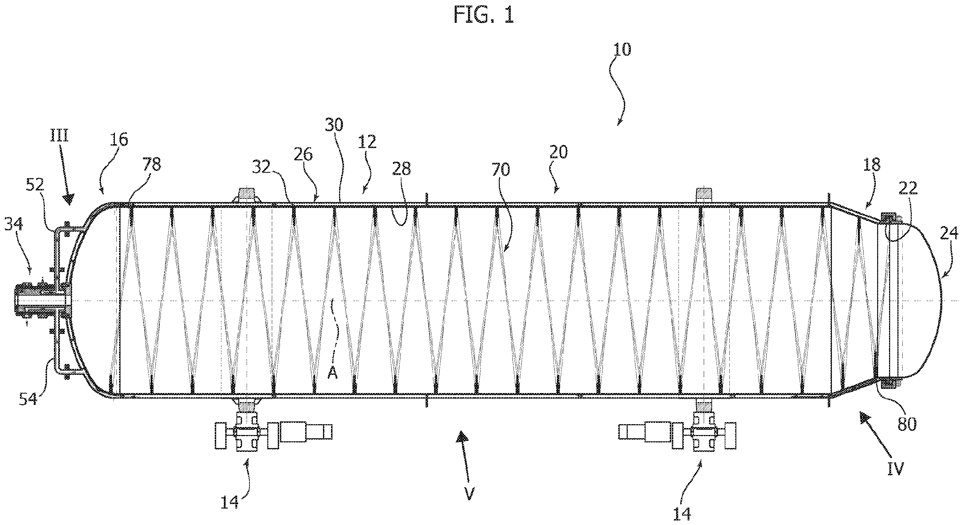

With reference to Figure 1, numeral 10 indicates an apparatus for sterilizing post-consumer absorbent sanitary products.

The apparatus comprises a container 12 rotatably supported about a longitudinal axis A by means of stationary supports 14, at least one of which is provided with actuating means that rotate the container 12 about the axis A. The longitudinal axis A of the container 12 is kept constantly fixed, preferably in a horizontal position.

The container 12 has an elongated shape along the longitudinal axis A, with an elongated cylindrical body 20 that extends between a first end 16 and a second end 18. The first end 16 is closed and has a generally hemispherical shape, and the second end 18 is provided with a loading/unloading opening 22, which is sealed closed by a removable door 24.

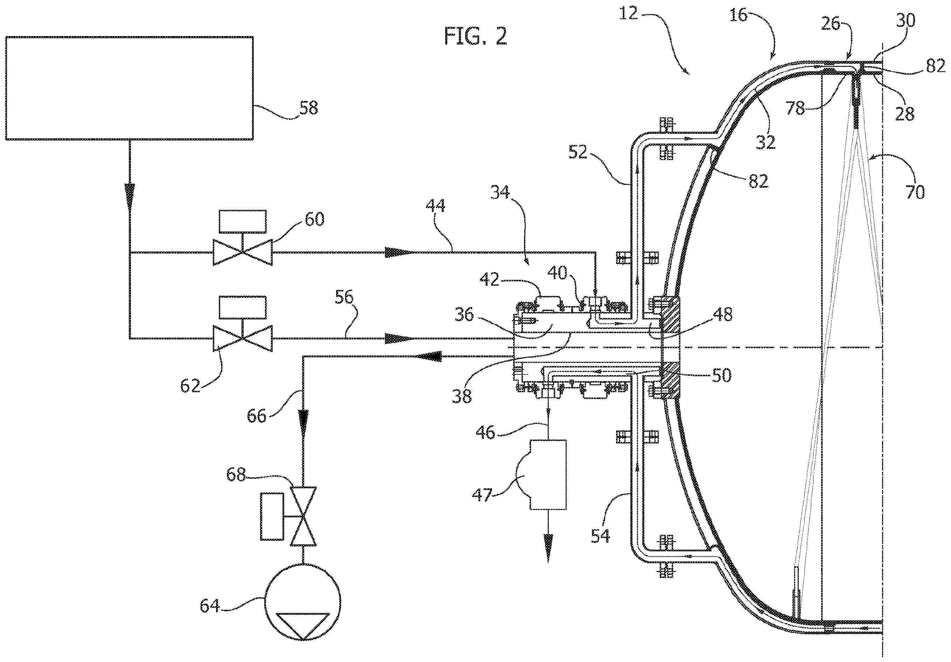

With reference in particular to Figures 2-5, the container 12 has a hollow wall 26 including an inner wall 28 and an outer wall 30, which enclose a hollow space 32. The hollow space 32 extends along the entire cylindrical body 20, from the first end 16 to the second end 18. Preferably, the hollow space also extends on the closed end 16. The inner wall 28 of the container 12 has an inner surface that delimits a treatment volume.

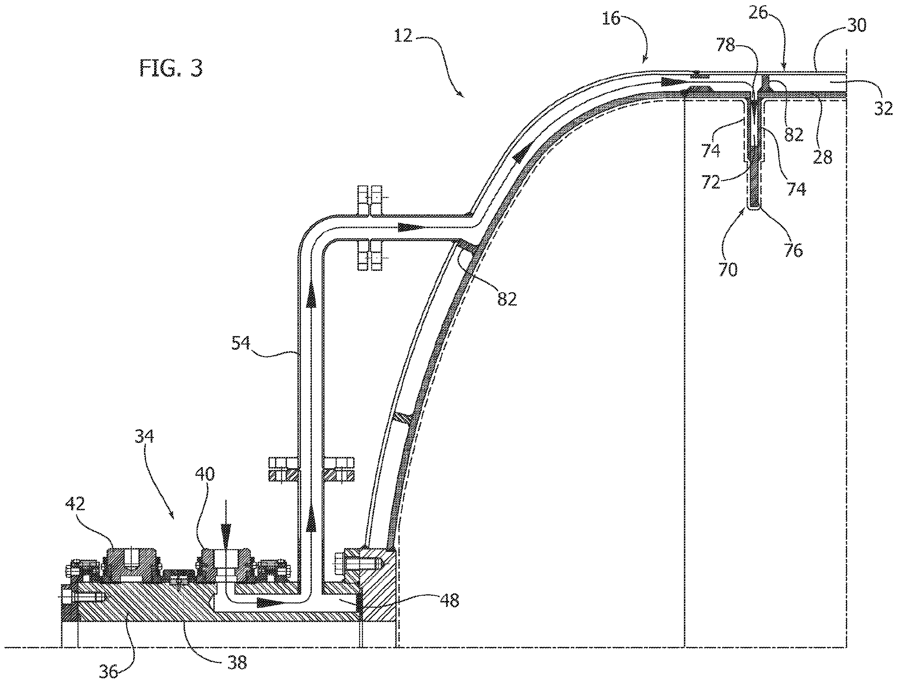

With reference in particular to Figures 2 and 3, the container 12 comprises a rotary joint 34 located at the first end 16 coaxial with the longitudinal axis A. The rotary joint 34 comprises a hub 36 fixed to the wall 26 of the container 12, having a hole 38 in communication with the inside of the container 12. The rotary joint 34 comprises two stationary rings 40, 42 arranged in sliding sealed contact with the central hub 36.

With reference to Figure 2, the stationary rings 40, 42 are connected, respectively, to a steam supply pipe 44 and to a condensate discharge pipe 46. An automatic condensate discharge device 47 is arranged on the condensate discharge pipe 46. In the central hub 36, a steam inlet chamber 48 and a condensate discharge chamber 50 are formed, in fluid connection with the respective stationary rings 40, 42. The steam inlet chamber 48 and the condensate discharge chamber 50 are connected to respective portions of the hollow space 32 through respective pipes 52, 54.

The hole 38 of the hub 36 is connected to a second steam supply pipe 56, which supplies pressurized steam inside the container 12 in direct contact with the products (contact steam). The steam supply pipes 44, 56 are connected to a steam generator 58 by means of respective valves 60, 62. The hole 38 of the hub 36 is also connected to a vacuum pump 64 via a pipe 66 and a valve 68. The vacuum pump 64 extracts air from the container 12 prior to pressurization of the container 12 with the contact steam.

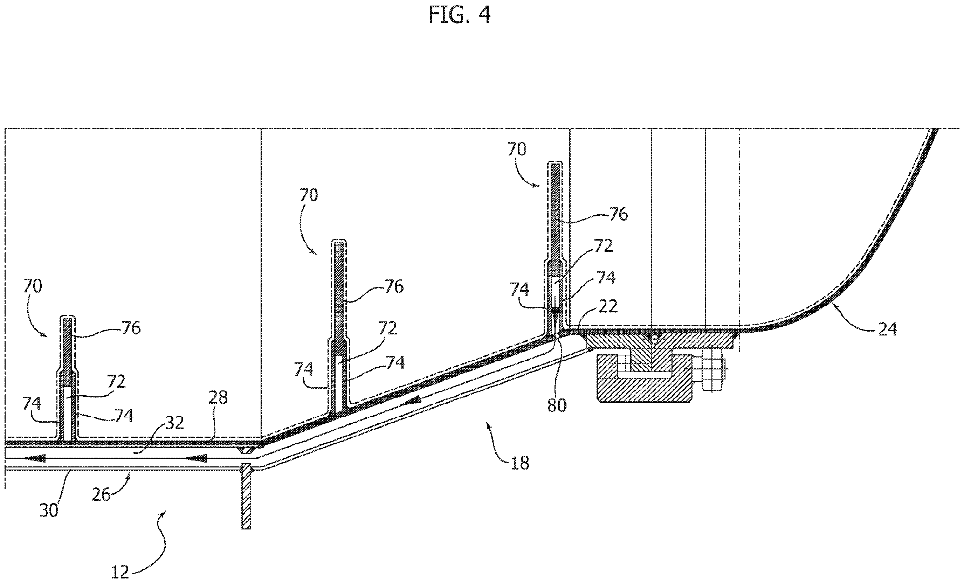

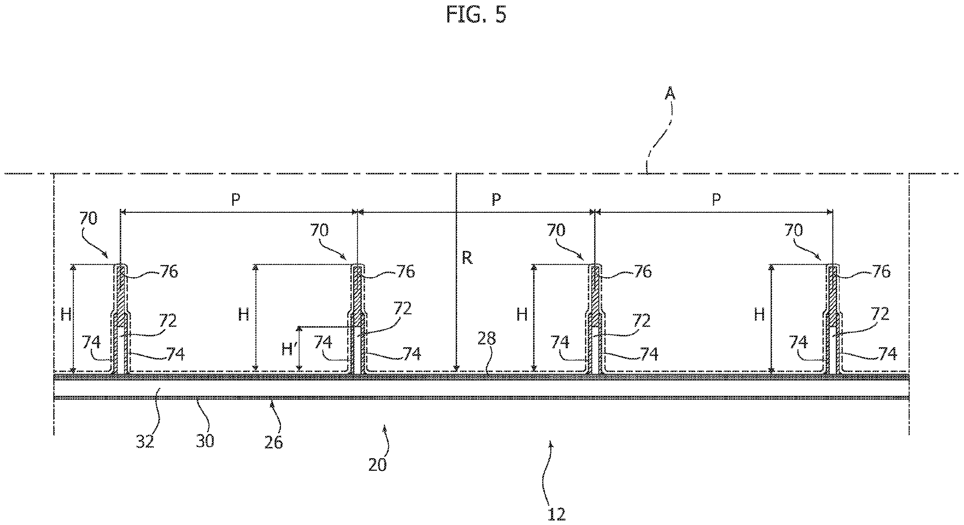

The container 12 has at least one hollow helical blade 70 fixed to the inner wall 28 of the container 12 and projecting inwardly of the container 12. The hollow helical blade 70 forms a screw with a pitch P (Figure 5), coaxial with the longitudinal axis A and extending in the longitudinal direction A between the first end 16 and the second end 18 of the container 12. With reference to Figures 4 and 5, the hollow helical blade 70 has a helical cavity 72 which extends along the entire length of the hollow helical blade 70. The helical cavity 72 is delimited laterally by two first helical plates 74. A third helical plate 76 is fixed between the first two helical plates 74 and projects inwardly of the container 12 beyond the distal edges of the first two helical plates 74. The helical cavity 72 is delimited along its minor sides by an edge of the third helical plate 76 and by a part of the inner wall 28 of the container 12.

In the example illustrated in the Figures, the first two helical plates 74 are parallel to each other, so that the helical cavity 72 has a cross-section in the shape of a narrow rectangle. In other embodiments, the first two helical plates 74 could be inclined with respect to one another, so that the helical cavity 72 would have a triangular shape in cross-section.

With reference to Figure 5, the ratio between the height H of the hollow helical blade 70 and the radius R of the inner wall 28 of the container 12 is preferably equal to 0.2 ± 10%. The ratio between the height H' of the cavity 72 and the height H of the hollow helical blade 70 is preferably equal to 0.4 ± 10%.

With reference to Figures 3 and 4, the helical cavity 72 has an inlet opening 78 (Figure 3) located at the first end 16 of the container 12 and an outlet opening 80 (Figure 4) located at the second end 18 of the container 12. The inlet opening 78 and the outlet opening 80 are open on the hollow space 32. With reference to Figure 3, the portion of the hollow space 32 comprised between the outlet of the pipe 54 and the inlet opening 78 of the helical cavity 72 is closed by septa 82, so that the inlet opening 78 communicates directly with the steam inlet chamber 48 of the rotating connector 34 via a portion of the hollow space 32 and by means of the pipe 52. Alternatively, the outlet of the pipe 52 could be directly connected to the inlet opening 78 of the helical cavity 72 without a portion of closed hollow space between the outlet of the pipe 52 and the inlet opening 78.

The operation of the apparatus 10 is as follows.

The container 12 is loaded with a load of post-consumer absorbent sanitary products coming from the recycling collection. Typically, absorbent sanitary products comprise an absorbent core of cellulose fibers and superabsorbent polymers. The absorbent core is usually enclosed between two sheets of plastic material joined together. Typically, the outer sheet (backsheet) is impermeable while the inner sheet (topsheet) is porous. The used absorbent sanitary products are usually folded so as to close the product in a packet form inside the waterproof outer sheet. Adhesive wings are usually provided to close the folded product. Organic excretions are thus enclosed within a sealed waterproof plastic sheet. The closed absorbent sanitary products are usually contained in plastic bags of the type normally used to contain household waste. The absorbent sanitary products are subjected to the sterilization treatment as they come from the collection.

The bags containing the absorbent sanitary products are loaded into the container 12 through the opening 22 after removing the door 24. During loading of the products, the container is rotated so that the helical blade 72 transports the products from the second end 18 towards the first end 16. When the container has been loaded with a predetermined amount of products (which can, for example, be measured with a load cell), the door 24 is closed and the vacuum pump 64 is activated to extract the air from the treatment volume. Then, the treatment volume is filled with pressurized contact steam fed through the hole 38 of the rotary joint 34.

At the same time, the wall 26 of the container 12 and the hollow helical blade 70 are heated by non-contact steam. The non-contact steam is fed from the chamber 48 of the rotary joint 34 to the inlet opening 78 of the hollow helical blade 70. Non-contact steam travels through the helical cavity 72 of the hollow helical blade 70. At the outlet 80 of the helical cavity 72, the non-contact steam fills the hollow space 32 of the wall 26. The condensed steam is expelled through the condensate discharge pipe 54 and the automatic condensate discharge device 47.

During the sterilization treatment, the container 12 is rotated about the axis A alternately in opposite directions. In this way, the products are continuously moved and are transported alternately in opposite directions inside the container 12. The non-contact steam inside the hollow helical blade 70 and the hollow space 26 creates hot spots that cause the bags and the outer plastic sheets of the absorbent sanitary products to break. These hot spots create localized fusion points that open holes in the plastic sheets and weaken the outer casings. The continuous movement of the products caused by the rotation of the container and of the helical blade 70 causes breakage of the outer casings and destructuring of the products. This allows a more effective drying and separation action of the materials.

The alternate transport of the products from the first end 16 to the second end 18 of the container 12, and vice versa, by means of the heated helical blade 70 provides effective contact of the products with the helical blade 70 and improves the transfer of heat to the products.

A particularly advantageous aspect of the present invention is that the non-contact steam first crosses the helical blade 70 and subsequently the hollow space 32 of the wall 26, so that the hollow helical blade 70 is hotter than the wall 26. Thus, the mass of products furthest from the wall 26, which receives less heat from the contact with the wall 26, receives more heat from the contact with the helical blade 70. This provides a more efficient and uniform heat transfer from the non-contact steam to the products.

Of course, without prejudice to the principle of the invention, the details of construction and the embodiments can be widely varied with respect to those described and illustrated, without thereby departing from the scope of the invention as defined by the claims that follow.

## Claims

1. A sterilizing apparatus for sterilizing post-consumer absorbent sanitary products, comprising:
- a container (12) rotatable about a longitudinal axis (A), having a closed first end (16) and a second end (18) provided with a loading/unloading opening (22) closed by a removable door (24), wherein the container (12) has a hollow wall (26) including an inner wall (28), an outer wall (30), and a hollow space (32), which extends from said first end (16) to said second end (18),
- a rotary joint (34) located at said first end (16) of the container (12) and having a steam inlet chamber (48) and a condensate discharge chamber (50), connected to respective portions of said hollow space (32) through respective pipes (52, 54),
- at least one hollow helical blade (70), which extends between said first end (16) and said second end (18) of the container (12) and protrudes inwardly from said inner wall (28) of the container (12), wherein said hollow helical blade (70) has a helical cavity (72) having an inlet (78) in fluid communication with said steam inlet chamber (48) at said first end of the container (16), an outlet (80) in fluid communication with said hollow space (32) at said second end (18) of the container (12), and is closed between said inlet (78) and said outlet (80), wherein a portion of said hollow space (32) comprised between the outlet of one of said pipes (54) and the inlet opening (78) of the helical cavity (72) is closed by septa (82), so that said inlet opening (78) communicates directly with the steam inlet chamber (48) of the rotating connector (34) via a portion of said hollow space (32) and by means of one of said pipes (52),
wherein the condensate discharge chamber (50) of said rotary joint (34) is in fluid communication with said hollow space (32) in said first end (16) of the container (12), so that the steam coming from said steam inlet chamber (48) flows into said helical cavity (72) from said first end (16) towards said second end (18) and, subsequently, flows into said hollow space (32) from said second end (18) toward said first end (16) .

2. An apparatus according to claim 1, wherein said inner wall (26) has a radius (R) with respect to said rotation axis, wherein said helical hollow blade has a height (H), and wherein the ratio between said height (H) and said radius (R) is equal to 0.2 ± 10%.

3. An apparatus according to claim 2, wherein said helical cavity (72) has a height (H'), and wherein the ratio between said height of the cavity (H') and said height (H) of the hollow helical blade (70) is equal to 0.4 ± 10%.

4. An apparatus according to claim 1, wherein said wall (26) has a cylindrical shape between said first end (26) and said second end (18).

5. An apparatus according to claim 1, wherein said longitudinal axis (A) has a fixed orientation.

6. An apparatus according to claim 1, wherein said hollow helical blade (70) carries out transportation of the products within the container (12) from said first end (16) towards said second end (18), and vice versa, following rotation of the container (12) about said longitudinal axis (A).

## Patentansprüche

1. Sterilisationsvorrichtung zum Sterilisieren von gebrauchten Absorptionshygieneartikeln, umfassend:
- einen Behälter (12), der um eine Längsachse (A) herum drehbar ist, der ein geschlossenes erstes Ende (16) und ein zweites Ende (18) aufweist, der mit einer Be-/Entladeöffnung (22) versehen ist, die durch eine entfernbare Klappe (24) verschlossen ist, wobei der Behälter (12) eine hohle Wand (26) aufweist, die eine Innenwand (28), eine Außenwand (30) und einen hohlen Raum (32) einschließt, der sich von dem ersten Ende (16) zu dem zweiten Ende (18) erstreckt,
- ein Drehgelenk (34), das sich an dem ersten Ende (16) des Behälters (12) befindet und eine Dampfeinlasskammer (48) und eine Kondensatabführkammer (50) aufweist, die mit jeweiligen Abschnitten des hohlen Raums (32) durch jeweilige Rohre (52, 54) verbunden sind,
- mindestens eine hohle spiralförmige Klinge (70), die sich zwischen dem ersten Ende (16) und dem zweiten Ende (18) des Behälters (12) erstreckt und von der Innenwand (28) des Behälters (12) nach innen vorsteht, wobei die hohle spiralförmige Klinge (70) einen spiralförmigen Hohlraum (72) aufweist, der einen Einlass (78) in Fluidaustausch mit der Dampfeinlasskammer (48) an dem ersten Ende des Behälters (16) aufweist, wobei ein Auslass (80) mit dem hohlen Raum (32) an dem zweiten Ende (18) des Behälters (12) in Fluidaustausch steht und zwischen dem Einlass (78) und dem Auslass (80) geschlossen ist, wobei ein Abschnitt des hohlen Raums (32), der zwischen dem Auslass eines der Rohre (54) und der Einlassöffnung (78) des spiralförmigen Hohlraums (72) besteht, durch Septen (82) geschlossen ist, sodass die Einlassöffnung (78) mit der Dampfeinlasskammer (48) des sich drehenden Verbinders (34) über einen Abschnitt des hohlen Raums (32) und mittels eines der Rohre (52) direkt verbunden ist,
wobei die Kondensatabführkammer (50) des Drehgelenks (34) in Fluidaustausch mit dem hohlen Raum (32) in dem ersten Ende (16) des Behälters (12) steht, sodass der Dampf, der aus der Dampfeinlasskammer (48) kommt, in den spiralförmigen Hohlraum (72) von dem ersten Ende (16) zu dem zweiten Ende (18) hin strömt und anschließend in den hohlen Raum (32) von dem zweiten Ende (18) zu dem ersten Ende (16) hin strömt.

2. Vorrichtung nach Anspruch 1, wobei die Innenwand (26) einen Radius (R) in Bezug auf die Drehachse aufweist, wobei die spiralförmige hohle Klinge eine Höhe (H) aufweist und wobei das Verhältnis zwischen der Höhe (H) und dem Radius (R) gleich 0.2 ± 10 % ist.

3. Vorrichtung nach Anspruch 2, wobei der spiralförmige Hohlraum (72) eine Höhe (H') aufweist und wobei das Verhältnis zwischen der Höhe des Hohlraums (H') und der Höhe (H) der hohlen spiralförmigen Klinge (70) 0,4 ± 10 % beträgt.

4. Vorrichtung nach Anspruch 1, wobei die Wand (26) eine zylindrische Form zwischen dem ersten Ende (26) und dem zweiten Ende (18) aufweist.

5. Vorrichtung nach Anspruch 1, wobei die Längsachse (a) eine feste Ausrichtung aufweist.

6. Vorrichtung nach Anspruch 1, wobei die hohle spiralförmige Klinge (70) einen Transport der Artikel innerhalb des Behälters (12) von dem ersten Ende (16) zu dem zweiten Ende (18) und umgekehrt nach der Drehung des Behälters (12) um die Längsachse (A) herum durchführt.

## Revendications

1. Appareil de stérilisation permettant de stériliser des produits hygiéniques absorbants post-consommation, comprenant :
- un récipient (12) rotatif autour d'un axe longitudinal (A), ayant une première extrémité fermée (16) et une seconde extrémité (18) pourvue d'une ouverture de chargement/déchargement (22) fermée par une porte amovible (24), dans lequel le récipient (12) a une paroi creuse (26) comportant une paroi interne (28), une paroi externe (30), et un espace creux (32), qui s'étend de ladite première extrémité (16) à ladite seconde extrémité (18),
- une articulation rotative (34) située au niveau de ladite première extrémité (16) du récipient (12) et ayant une chambre d'entrée de vapeur (48) et une chambre de décharge de condensat (50), raccordées à des parties respectives dudit espace creux (32) à travers des tuyaux respectifs (52, 54),
- au moins une lame hélicoïdale creuse (70), qui s'étend entre ladite première extrémité (16) et ladite seconde extrémité (18) du récipient (12) et fait saillie vers l'intérieur à partir de ladite paroi interne (28) du récipient (12), dans lequel ladite lame hélicoïdale creuse (70) a une cavité hélicoïdale (72) ayant une entrée (78) en communication du point de vue des fluides avec ladite chambre d'entrée de vapeur (48) au niveau de ladite première extrémité du récipient (16), une sortie (80) en communication du point de vue des fluides avec ledit espace creux (32) au niveau de ladite seconde extrémité (18) du récipient (12), et est fermée entre ladite entrée (78) et ladite sortie (80), dans lequel une partie dudit espace creux (32) comprise entre la sortie de l'un desdits tuyaux (54) et l'ouverture d'entrée (78) de la cavité hélicoïdale (72) est fermée par des septums (82), de sorte que ladite ouverture d'entrée (78) communique directement avec la chambre d'entrée de vapeur (48) du raccord rotatif (34) par l'intermédiaire d'une partie dudit espace creux (32) et au moyen d'un desdits tuyaux (52),
dans lequel la chambre de décharge de condensat (50) de ladite articulation rotative (34) est en communication du point de vue des fluides avec ledit espace creux (32) dans ladite première extrémité (16) du récipient (12), de sorte que la vapeur provenant de ladite chambre d'entrée de vapeur (48) s'écoule dans ladite cavité hélicoïdale (72) à partir de ladite première extrémité (16) vers ladite seconde extrémité (18) et, ultérieurement, s'écoule dans ledit espace creux (32) à partir de ladite seconde extrémité (18) vers ladite première extrémité (16).

2. Appareil selon la revendication 1, dans lequel ladite paroi interne (26) a un rayon (R) par rapport audit axe de rotation, dans lequel ladite lame creuse hélicoïdale a une hauteur (H), et dans lequel le rapport entre ladite hauteur (H) et ledit rayon (R) est égal à 0,2 ± 10 %.

3. Appareil selon la revendication 2, dans lequel ladite cavité hélicoïdale (72) a une hauteur (H'), et dans lequel le rapport entre ladite hauteur de la cavité (H') et ladite hauteur (H) de la lame hélicoïdale creuse (70) est égal à 0,4 ± 10 %.

4. Appareil selon la revendication 1, dans lequel ladite paroi (26) a une forme cylindrique entre ladite première extrémité (26) et ladite seconde extrémité (18).

5. Appareil selon la revendication 1, dans lequel ledit axe longitudinal (A) a une orientation fixe.

6. Appareil selon la revendication 1, dans lequel ladite lame hélicoïdale creuse (70) effectue le transport des produits au sein du récipient (12) à partir de ladite première extrémité (16) vers ladite seconde extrémité (18), et vice versa, suite à la rotation du récipient (12) autour dudit axe longitudinal (A).
